# EUROPEAN PATENT APPLICATION

(11) **EP 3 424 513 A1**
(43) Date of publication of application: **09.01.2019**
(21) Application number: 17759722.6
(22) Date of filing: 20.02.2017
(51) Int. Cl.: A61K 31/787, A61K 9/19, A61K 31/4745, A61K 35/00, A61K 47/02, A61K 47/18, A61K 47/34, A61K 47/42, A61K 47/50

(54) **PHARMACEUTICAL PREPARATION CONTAINING CAMPTOTHECIN-BASED POLYMERIC DERIVATIVE**

(30) Priority: 01.03.2016 JP 2016038943
(71) Applicant: Nippon Kayaku Kabushiki Kaisha, Tokyo 100-0005 (JP)
(72) Inventor: AOKI Shin, Tokyo 115-8588 (JP); FUJITA Shinya, Tokyo 115-8588 (JP)
(74) Representative: Ter Meer Steinmeister & Partner
(86) International application number: PCT/JP2017/006192
(87) International publication number: WO 2017/150256

(57) **Abstract**

Provided is a pharmaceutical preparation including a polymerized camptothecin derivative obtained by bonding a camptothecin derivative to a polymer carrier, the polymerized camptothecin derivative being capable of associating in an aqueous solution and having a nanoparticle-forming property, and the pharmaceutical preparation is a pharmaceutical preparation composition having enhanced preparation stability. Particularly, a pharmaceutical preparation having an excellent associated aggregate-forming property, which is an important factor, and excellent storage stability against chemical stability is provided. Disclosed is a pharmaceutical preparation including a block copolymer in which a polyethylene glycol segment and a polyglutamic acid segment containing a glutamic acid unit having a camptothecin derivative bonded thereto, and a pharmaceutical preparation including one or more additives selected from the group consisting of arginine, histidine, and sodium chloride.

## Description

### TECHNICAL FIELD

The present invention relates to a pharmaceutical preparation having enhanced preparation stability, the pharmaceutical preparation including a polymerized camptothecin derivative having a camptothecin derivative bonded to a polymer carrier. The polymerized camptothecin derivative has associative properties between a plurality of the derivative molecules in an aqueous solution and has a property of forming nanoparticles. The invention relates to a technology concerning a pharmaceutical preparation having excellent storage stability, the pharmaceutical preparation including a polymerized camptothecin derivative having such a nanoparticle-forming property.

### BACKGROUND ART

In order to effectively exhibit the efficacy of a pharmaceutical product, it is required to cause a pharmacologically active compound to act on an appropriate site in vivo at an appropriate concentration at an appropriate time. Particularly, cytotoxic antitumor agents are widely distributed over the whole body and exhibit cell proliferation inhibitory action when systemically administered by intravenous administration or the like. At this time, since the pharmacologically active action is exhibited without any distinction between cancer cells and normal cells, it is considered that serious side effects are caused as a result of the action on normal cells. Therefore, in order to reduce side effects, a technology for delivering an antitumor agent to an affected site of tumor. Thus, there is a demand for a method of controlling the pharmacokinetics in order to selectively deliver an antitumor agent to a tumor tissue and to cause the antitumor agent to act at an appropriate drug concentration and at an appropriate drug sensitization time.

As a method for controlling the pharmacokinetics, a method of utilizing the pharmacokinetic characteristics based on the molecular weight is known. That is, when a biocompatible polymeric substance is administered into blood, renal excretion is suppressed, and the half-life in blood is maintained long. Furthermore, since tumor tissues have high tissue permeability of polymeric substances and the recovery mechanism for the polymeric substances has not been sufficiently established, polymeric substances are known to be distributed and accumulate at relatively high concentrations in tumor tissues. Thus, development of polymerized antitumor agents each having an antitumor agent bonded to a biocompatible polymeric substance has been attempted.

As polymerized antitumor agents, polymerized antitumor agents in which a block copolymer of a polyethylene glycol segment and a polyglutamic acid segment linked together is used as a polymer carrier, and various antitumor agents are bonded to the side-chain carboxylic acid of the polyglutamic acid segment, have been reported. Patent Literature 1 discloses a pharmaceutical product having 7-ethyl-10-hydroxycamptothecin bonded to the block copolymer. Furthermore, as other antitumor agents, a block copolymer conjugate of a cytidine-based antitumor agent (Patent Literature 2), a block copolymer conjugate of combretastatin A4 (Patent Literature 3), a block copolymer conjugate of an HSP90 inhibitor (Patent Literature 4), and the like are known. It is described that these polymerized antitumor agents have enhanced antitumor effects, compared to low molecular weight antitumor compounds that are used as active ingredients.

These block copolymer conjugates of antitumor agents are polymerized antitumor agents a hydroxy group of the antitumor agent and a side-chain carboxylic acid of the block copolymer are bonded by an ester bond. These polymerized antitumor agents are prodrugs that exhibit antitumor active action as the ester bond is cleaved at a certain rate to release the antitumor agent when the polymerized antitumor agent is administered in vivo.

Furthermore, these block copolymers having antitumor agents bonded thereto have a property in which when the block region to which the antitumor agent is bonded is hydrophobic, the antitumor agent-bonded region shows associative properties based on a hydrophobic interaction in an aqueous solution, and a plurality of the block copolymer molecules form associated aggregates.

Regarding these associated aggregates, the physical properties of the associated aggregates may be measured by light scattering analysis using laser light or the like. That is, the physical properties of the associated aggregates may be specified by taking the light scattering intensity as a measured value.

These polymerized antitumor agents exhibit a high antitumor effect, as the molecules behave as nanoparticles in vivo and are distributed at high concentrations in tumor tissues by exhibiting pharmacokinetics as described above, and the polymerized antitumor agents release the antitumor agents there. Therefore, in these polymerized antitumor agents, the property of forming nanoparticles is an important factor for exhibiting the performance of the agents.

A drug-polymer conjugate pharmaceutical product is a pharmaceutical product with which high pharmacological activity and reduction of side effects are promoted by means of the pharmacokinetics based on the molecular weight of the polymer carrier and slow release of the conjugated drug as an active form. Therefore, such a drug-polymer conjugate pharmaceutical product needs to be prepared into a preparation having reduced changes in the molecular weight of the polymer carrier in a state of being stored as a preparation, that is, a preparation having excellent storage stability with suppressed reduction of molecular weight.

In regard to drug-polymer conjugate pharmaceutical products, as preparations considering storage stability, for example, Patent Literatures 5 and 6 disclose that when a conjugate of a polysaccharide having carboxy groups and a camptothecin derivative is prepared into a pharmaceutical preparation including a sugar or a sugar alcohol and a pH adjusting agent, molecular weight change of the polymer carrier or release of the camptothecin derivative is suppressed.

It is thought that these drug-polymer conjugate pharmaceutical products do not form associated bodies, and it is conceived that the molecular weight of the polymer carrier is a performance-exhibiting factor. For this reason, molecular weight reduction by a chemical decomposition reaction such as cleavage of chemical bonds of the carrier becomes a problem, and suppression thereof is needed. However, in regard to a polymerized antitumor agent based on a block copolymer, in which polymerization by nanoparticle formation by associated aggregates serves as a performance management factor (performance exhibiting factor), a stable pharmaceutical preparation intended to have the nanoparticle-forming ability well-controlled is not known.

### CITATION LIST

### PATENT LITERATURE

Patent Literature 1: WO 2004/39869 A
Patent Literature 2: WO 2008/056596 A
Patent Literature 3: WO 2008/010463 A
Patent Literature 4: WO 2008/041610 A
Patent Literature 5: WO 2002/005855 A
Patent Literature 6: JP 2005-523329 A

### SUMMARY OF INVENTION

### TECHNICAL PROBLEM

It is an object of the present invention to provide a pharmaceutical preparation of a polymerized camptothecin derivative having a camptothecin derivative bonded to a polymer carrier, the pharmaceutical preparation having controlled associated aggregate-forming property and having enhanced preparation stability. Furthermore, it is an object of the invention to provide a pharmaceutical preparation having excellent chemical stability, in which dissociation of a camptothecin derivative bonded to a polymer carrier from the carrier is suppressed.

It is an object of the invention to provide a pharmaceutical preparation including a polymerized camptothecin derivative having such nanoparticle-forming property, the pharmaceutical preparation having excellent storage stability, in which the nanoparticle-forming property, which is an important factor of the pharmaceutical preparation performance, are maintained and the production of decomposition products is suppressed.

### SOLUTION TO PROBLEM

The inventors of the present invention found that, by using a particular additive together with a polymerized camptothecin derivative which is a block copolymer having a polyethylene glycol segment linked to a polyglutamic acid segment containing a glutamic acid unit having a camptothecin derivative bonded thereto, a pharmaceutical preparation that maintains a property to form nanoparticles by forming the associated aggregates stably for a long time period and has excellent storage stability with suppressed release of the camptothecin derivative, is obtained. Thus, the inventors completed the invention. The present application is mainly related to the following inventions.
[1] A pharmaceutical preparation including:
   a block copolymer of a polyethylene glycol segment and a polyglutamic acid segment linked together, the polyglutamic acid segment containing a glutamic acid unit having a camptothecin derivative bonded thereto; and
   one or more additives selected from the group consisting of arginine, histidine, and sodium chloride,
   wherein the block copolymer is a block copolymer represented by General Formula (1): 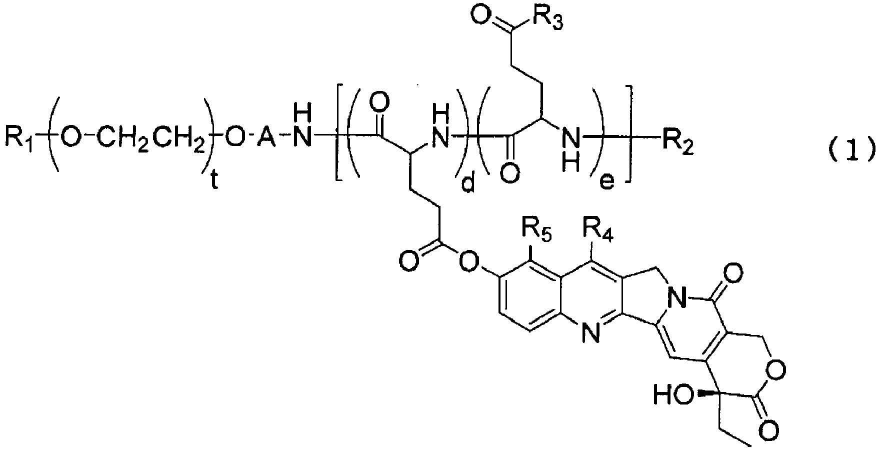 wherein
      **R**₁ represents a hydrogen atom or an optionally substituted (C1-C6) alkyl group;
      **A** represents a (C1-C6) alkylene group;
      **R**₂ represents any one selected from the group consisting of a hydrogen atom, an optionally substituted (C1-C6) acyl group, and an optionally substituted (C1-C6) alkoxycarbonyl group;
      **R**₃ represents a hydrogen group and/or -N(**R**₆)CONH(**R**₇);
         **R**₆ and **R**₇, which may be identical or different, each represent a (C1-C8) alkyl group which may be substituted with a tertiary amino group;
      **R**₄ represents any one selected from the group consisting of a hydrogen atom, an optionally substituted (C1-C6) alkyl group, and an optionally substituted silyl group;
      **R**₅ represents a hydrogen atom or an optionally substituted (C1-C6) alkyl group;
      **t** represents an integer from 45 to 450;
      **d** and **e** each represent an integer; **d** + **e** represents an integer from 6 to 60; the proportion of **d** with respect to **d** + **e** is 1% to 100%; the proportion of **e** is 0% to 99%; and
      the polyglutamic acid segment has a polyglutamic acid segment structure in which glutamic acid units having a camptothecin derivative bonded thereto and glutamic acid units having a **R**₃ group bonded thereto are each independently arranged randomly.

   The pharmaceutical preparation of the present invention may stably maintain the associated aggregate-forming property of the block copolymer during storage. Furthermore, a pharmaceutical preparation having excellent chemical stability with suppressed dissociation of the camptothecin derivative bonded to the polymer carrier may be provided.
[2] The pharmaceutical preparation described in [1], wherein the pharmaceutical preparation is a freeze-dried preparation.

Since the present invention is a preparation form that may have the nanoparticle-forming prepared into a freeze-dried preparation as the preparation form, a preparation form that is desirable from the viewpoint of stably controlling and easily maintaining the nanoparticle-forming property is provided. Furthermore, when the invention is prepared into a freeze-dried preparation, it is more desirable from the viewpoint that the dissolution rate may be increased at the time of reconstituting the pharmaceutical preparation into an aqueous solution.

### ADVANTAGEOUS EFFECTS OF INVENTION

In regard to the block copolymer according to the present invention, in which a polyethylene glycol segment is linked to a polyglutamic acid segment containing a glutamic acid unit having a camptothecin derivative bonded thereto, nanoparticle formation by associated aggregates is an essential performance for exhibiting efficacy, and in particular, it is important to be able to form nanoparticles in a desired association state. Furthermore, it is also important that the amount of decomposition products prepared during storage is reduced.

The pharmaceutical preparation of the present invention may provide a pharmaceutical preparation including the block copolymer that forms associated aggregates as an active ingredient, the pharmaceutical preparation having excellent storage stability. That is, a pharmaceutical preparation having excellent physical stability and chemical stability, in which a desired association state is maintained during storage of the pharmaceutical preparation, or release of a camptothecin derivative resulting from dissociation of the derivative is reduced, may be provided.

### DESCRIPTION OF EMBODIMENTS

In the present invention, on the occasion of preparing a preparation having excellent storage stability and including a block copolymer having a polyethylene glycol segment linked to a polyglutamic acid segment containing a glutamic acid unit having a camptothecin derivative bonded thereto as an active ingredient, when histidine, arginine, or sodium chloride is used as an additive in the pharmaceutical preparation, a pharmaceutical preparation including the block copolymer having excellent storage stability, in which after the pharmaceutical preparation was stored under light-shielded conditions for four weeks at 40°C, the change ratio of the formation of associated aggregates of the pharmaceutical preparation and the production amount of the released camptothecin derivative are low, and the block copolymer having excellent storage stability is included, could be prepared. The present invention will be explained in detail below.

The present invention uses a block copolymer in which a polyethylene glycol segment is linked to a polyglutamic acid segment containing a glutamic acid unit having a camptothecin derivative, the block copolymer being represented by the following General Formula (1): 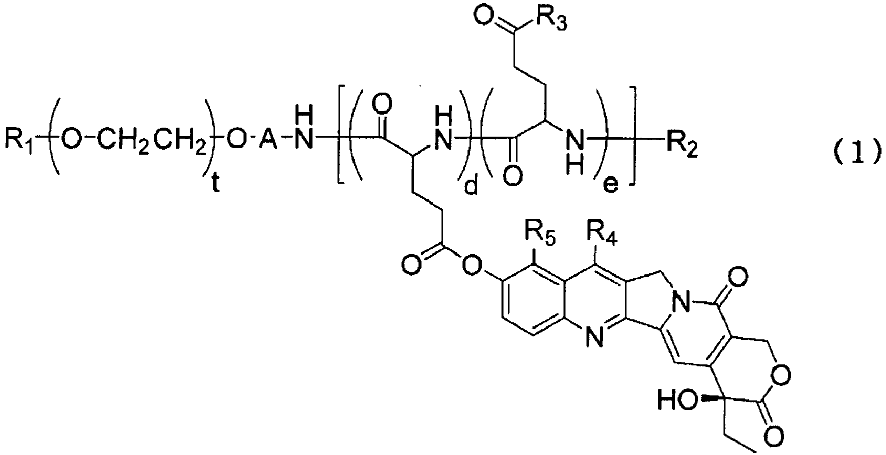 wherein
**R**₁ represents a hydrogen atom or an optionally substituted (C1-C6) alkyl group;
**A** represents a (C1-C6) alkylene group;
**R**₂ represents any one selected from the group consisting of a hydrogen atom, an optionally substituted (C1-C6) acyl group, and an optionally substituted (C1-C6) alkoxycarbonyl group;
**R**₃ represents a hydroxy group and/or N(**R**₆)CONH(**R**₇);
   **R**₆ and **R**₇, which may be identical or different, each represent a (C1-C8) alkyl group which may be substituted with a tertiary amino group;
**R**₄ represents any one selected from the group consisting of a hydrogen atom, an optionally substituted (C1-C6) alkyl group, and an optionally substituted silyl group;
**R**₅ represents a hydrogen atom or an optionally substituted (C1-C6) alkyl group;
**t** represents an integer from 45 to 450;
**d** and **e** each represent an integer, **d** + **e** represents an integer from 6 to 60, and the proportion of **d** with respect to **d** + **e** is 1% to 100%, while the proportion of **e** is 0% to 99%; and
the polyglutamic acid segment has a polyglutamic acid segment structure in which a glutamic acid unit having a camptothecin derivative bonded thereto and a glutamic acid unit having an **R**₃ group bonded thereto are each independently arranged randomly.

The block copolymer is a block copolymer in which a polyethylene glycol segment is linked to a polyglutamic acid segment containing a glutamic acid unit having a camptothecin derivative bonded to the side chain by an ester bond, via an appropriate linking group.

The optionally substituted (C1-C6) alkyl group for **R**₁ may be an optionally substituted, linear, branched or cyclic (C1-C6) alkyl group. Examples of such an alkyl group include a methyl group, an ethyl group, an n-propyl group, an isopropyl group, an n-butyl group, an s-butyl group, a t-butyl group, an n-propyl group, a neopentyl group, a cyclopentyl group, an n-hexyl group, and a cyclohexyl group.

Examples of a substituent that may be carried by the alkyl group include a mercapto group, a hydroxy group, a halogen atom, a nitro group, a cyano group, a carbocyclic or heterocyclic aryl group, an alkylthio group, an arylthio group, an alkylsulfinyl group, an arylsulfinyl group, an alkylsulfonyl group, an arylsulfonyl group, a sulfamoyl group, an alkoxy group, an aryloxy group, an acyloxy group, an alkoxycarbonyloxy group, a carbamoyloxy group, a substituted or unsubstituted amino group, an acylamino group, an alkoxycarbonylamino group, a ureido group, a sulfonylamino group, a sulfamoylamino group, a formyl group, an acyl group, a carboxy group, an alkoxycarbonyl group, a carbamoyl group, and a silyl group. The position of substitution on the aromatic ring may be the ortho-position, the meta-position, or the para-position. An amino group, a dialkylamino group, an alkoxy group, a carboxy group, and a formyl group are preferred.

Preferred examples of **R**₁ include a methyl group, an ethyl group, an n-propyl group, an isopropyl group, an n-butyl group, an s-butyl group, a t-butyl group, a benzyl group, a 2,2-dimethoxyethyl group, a 2,2-diethoxyethyl group, and a 2-formylethyl group. An unsubstituted linear, branched or cyclic (C1-C4) alkyl group is preferred. Particularly, a methyl group, an ethyl group, an n-propyl group, an isopropyl group, an n-butyl group, an s-butyl group, a t-butyl group, and the like are preferred.

In General Formula (1), the polyethylene glycol segment is represented by a unit repetition structure of ethyleneoxy group; (-OCH₂CH₂) group, and regarding the polyethylene glycol moiety in that segment, it is preferable to use a moiety having a molecular weight of 2 kilodaltons to 20 kilodaltons, and more preferably a moiety having a molecular weight of 4 kilodaltons to 15 kilodaltons. That is, **t** in General Formula (1), which is the number of the unit repetition structures of ethyleneoxy group; (-OCH₂CH₂) group, is an integer from 45 to 450. Preferably, **t** is an integer from 90 to 340. For the molecular weight of the polyethylene glycol segment, the peak top molecular weight that may be determined by a GPC method using polyethylene glycol standard products is used.

**A** in General Formula (1), which is a linking group that links the polyethylene glycol segment to the polyglutamic acid segment, is a (C1-C6) alkylene group. Examples include a methylene group, an ethylene group, a trimethylene group, a tetramethylene group, and a hexamethylene group. Among them, an ethylene group or a trimethylene group is preferred, and a trimethylene group is particularly preferred.

The polyglutamic acid segment of the polymer compound of the present invention represented by General Formula (1) has a structure in which a glutamic acid unit is polymerized in an α-amide bond form. However, in the glutamic acid-polymerized structure, glutamic acid units that are polymerized in a γ-amide bond form may also be partially included. In the polyglutamic acid segment, each glutamic acid unit may be an L-form or a D-form, and those forms may exist as a mixture.

The total number of glutamic acid units in General Formula (1) is represented by **d** + **e,** and the total number is an integer from 6 to 60. Preferably, **d** + **e** is 8 to 40. Therefore, although the average molecular weight of the polyglutamic acid segment depends on the structures and binding amounts of the camptothecin derivative and the **R**₃ group, the average molecular weight is 0.6 kilodaltons to 15 kilodaltons, and preferably 0.8 kilodaltons to 10 kilodaltons.

The total number of glutamic acid units in the polyglutamic acid segment may be determined by a method for calculating the number of glutamic acid units by ¹H-NMR, an amino acid analysis method, a method for acid-base titration of side-chain carboxy groups, or the like. It is preferable that the number of glutamic acid units that is determined by subjecting side-chain carboxy groups to an acid-base titration method using the polyglutamic acid segment prior to bonding of the camptothecin derivative and the **R**₃ group to the side chains, is employed.

The optionally substituted (C1-C6) acyl group for **R**₂ may be an optionally substituted, linear, branched or cyclic (C1-C6) acyl group, and examples include a formyl group, an acetyl group, a propionyl group, a butyryl group, and a valeryl group.

Regarding a substituent, a hydroxy group, a halogen atom, an amino group, an alkylamino group, a dialkylamino group, an alkoxy group, an aryl group, and the like may be included.

Preferred examples of the optionally substituted (C1-C6) acyl group for **R**₂ include a formyl group, an acetyl group, a trichloroacetyl group, a trifluoroacetyl group, a propionyl group, a pivaloyl group, a benzylcarbonyl group, and a phenethylcarbonyl group. An optionally substituted, linear, branched or cyclic (C1-C4) acyl group is preferred, and an acetyl group, a trichloroacetyl group, and a trifluoroacetyl group are preferred.

The optionally substituted (C1-C6) alkoxycarbonyl group for **R**₂ may be an optionally substituted, linear, branched or cyclic (C1-C6) alkoxycarbonyl group. Regarding a substituent, a hydroxy group, a halogen atom, an amino group, an alkylamino group, a dialkylamino group, an alkoxy group, an aryl group, and the like may be included.

Preferred examples of the optionally substituted (C1-C6) alkoxycarbonyl group for **R**₂ include a methoxycarbonyl group, an ethoxycarbonyl group, a t-butoxycarbonyl group, a benzyloxycarbonyl group, and a 9-fluorenylmethyloxycarbonyl group.

For the **R**₂, it is preferable to use a hydrogen atom or an optionally substituted, linear, branched or cyclic (C1-C4) acyl group. **R**₂ is particularly preferably a hydrogen atom, an acetyl group, a trichloroacetyl group, or a trifluoroacetyl group.

In General Formula (1), **R**₃ represents a hydroxy group and/or **-**N(**R**₆)CONH(**R**₇). That is, a glutamic acid unit having **R**₃ at the side-chain carboxy group is a glutamic acid unit having an unmodified side chain, and/or a glutamic acid unit having a urea derivative bonded to the side chain.

**R**₆ and **R**₇, which may be identical or different, each represent a linear, branched or cyclic (C1-C8) alkyl group which may be substituted with a tertiary amino group. Examples of the (C1-C8) alkyl group for **R**₆ and **R**₇ include a methyl group, an ethyl group, an n-propyl group, an isopropyl group, an n-butyl group, a t-butyl group, a cyclopropyl group, a cyclohexyl group, and an n-octyl group.

Examples of the linear, branched or cyclic (C1-C8) alkyl group which may be substituted with a tertiary amino group include a 2-dimethylaminoethyl group and a 3-dimethylaminopropyl group.

Preferred examples of **R**₆ and **R**₇ include an ethyl group, an isopropyl group, a cyclohexyl group and a 3-dimethylaminopropyl group. More preferably, **R**₆ and **R**₇ may both represent an isopropyl group, **R**₆ and **R**₇ may both represent a cyclohexyl group, or **R**₆ and **R**₇ may represent an ethyl group and a 3-dimethylaminopropyl group, respectively.

As will be described below, -N(**R**₆)CONH(**R**₇) for **R**₃ is a modifying group of the glutamic acid side-chain, which is produced as a side-product by using a carbodiimide-based condensing agent when the block copolymer conjugated with a camptothecin derivative of General Formula (1) is synthesized. Therefore, **R**₆ and **R**₇ are identical to the alkyl substituents of the carbodiimide-based condensing agent used. That is, when diisopropylcarbodiimide (DIPCI) is used as the carbodiimide condensing agent, both **R**₆ and **R**₇ become an isopropyl group. When 1-ethyl-3-(3-dimethylaminopropyl)carbodiimide hydrochloride (WSC) is used, **R**₆ and **R**₇ become mixed substituents of an ethyl group and a 3-dimethylaminopropyl group. In this case, there are an occasion in which **R**₆ is an ethyl group while **R**₇ is a 3-dimethylaminopropyl group, and an occasion in which the reverse applies. **R**₆ and **R**₇ may also represent a - N(**R**₆)CONH(**R**₇) group, in which these ethyl group and 3-dimethylaminopropyl group exist as a mixture in one molecule.

In regard to General Formula (1), it is also acceptable that **R**₃ is a hydroxy group. That is, the polyglutamic acid segment according to the present invention may contain a free-form glutamic acid unit, to which neither a camptothecin derivative nor the -N(**R**₆)CONH(**R**₇) group is bonded. When **R**₃ at the side-chain carboxylic acid in the glutamic acid unit is a hydroxy group, the glutamic acid unit is in a free acid form; however, the side-chain carboxylic acid may also be in the form of a salt that may be used as a pharmaceutical product, and the form of a salt of an alkali metal or an alkaline earth metal is also included in the present invention. Examples of the alkali metal or alkaline earth metal salt include a lithium salt, a sodium salt, a potassium salt, a magnesium salt, and a calcium salt. When the pharmaceutical preparation of the present invention is supplied as an anticancer agent for parenteral administration, the pharmaceutical preparation is prepared into a solution using a pharmaceutically acceptable solution. In that case, the embodiment of the free-form glutamic acid unit may adopt any arbitrary form of glutamic acid salt depending on the pH of the solution and the presence of a salt in the buffer solution or the like.

The block copolymer represented by General Formula (1) includes a camptothecin derivative that is ester-bonded to a side-chain carboxy group of the polyglutamic acid segment. The camptothecin derivative is a camptothecin derivative having a hydroxy group that is supplied to the ester bond at the 10-position, and also has a **R**₄ group at the 7-position and a **R**₅ group at the 9-position. **R**₄ and **R**₅ may both represent a hydrogen atom; however, it is preferable that any one of **R**₄ and **R**₅ is a substituent other than a hydrogen atom.

**R**₄ represents a hydrogen atom, an optionally substituted (C1-C6) alkyl group, or an optionally substituted silyl group.

The optionally substituted (C1-C6) alkyl group for **R**₄ may be an optionally substituted, linear, branched or cyclic (C1-C6) alkyl group. Regarding a substituent, a hydroxy group, a halogen atom, an amino group, an alkylamino group, a dialkylamino group, an alkoxy group, an aryl group, and the like may also be included. Examples of the optionally substituted (C1-C6) alkyl group for **R**₄ include a methyl group, an ethyl group, an n-propyl group, an isopropyl group, an n-butyl group, an s-butyl group, a t-butyl group, and a benzyl group. An optionally substituted, linear, branched or cyclic (C1-C4) alkyl group is preferred, and an ethyl group is particularly preferred.

Examples of the optionally substituted silyl group for **R**₄ include a trimethylsilyl group, a triethylsilyl group, a t-butyldimethylsilyl group, a triisopropylsilyl group, and a t-butyldiphenylsilyl group. A t-butyldimethylsilyl group is preferred.

**R**₄ is preferably a hydrogen atom or an unsubstituted (C1-C6) alkyl group. A hydrogen atom or an ethyl group is particularly preferred.

**R**₅ represents a hydrogen atom or an optionally substituted (C1-C6) alkyl group.

The optionally substituted (C1-C6) alkyl group for **R**₅ may be an optionally substituted, linear, branched or cyclic (C1-C6) alkyl group. Regarding a substituent, a hydroxy group, a halogen atom, an amino group, an alkylamino group, a dialkylamino group, an alkoxy group, an aryl group, and the like may be included. Examples of the optionally substituted (C1-C6) alkyl group for **R**₅ include a methyl group, an ethyl group, an n-propyl group, an isopropyl group, an n-butyl group, an s-butyl group, a t-butyl group, a benzyl group, and a dimethylaminomethyl group.

**R**₅ is preferably a hydrogen atom or a (C1-C6) alkyl group having an amino group. A hydrogen atom or a dimethylaminomethyl group is particularly preferred.

The camptothecin derivative bonded to General Formula (1) is preferably 7-ethyl-10-hydroxycamptothecin and/or nogitecan (9-dimethylaminomethyl-10-hydroxycamptothecin). That is, an embodiment in which a hydroxy group at the 10-position of 7-ethyl-10-hydroxycamptothecin having an ethyl group as **R**₄ and a hydrogen atom as **R**₅ is bonded to a side-chain carboxy group of a polyglutamic acid segment by an ester bond, is preferred. Alternatively, an embodiment in which a hydroxy group at the 10-position of nogitecan (9-dimethylaminomethyl-10-hydroxycamptothecin) having a hydrogen atom as **R**₄ and a dimethylaminomethyl group as **R**₅ forms an ester bond, is preferred. Particularly preferred is an embodiment in which a hydroxy group at the 10-position of 7-ethyl-10-hydroxycamptothecin having an ethyl group as **R**₄ and a hydrogen atom as **R**₅ is bonded to a side-chain carboxy group of a polyglutamic acid segment by an ester bond.

The block copolymer described in General Formula (1) of the present invention preferably includes a plurality of camptothecin derivatives. In that case, the camptothecin derivatives bonded to chains of one molecular of the block copolymer may be identical; or a mixture of a plurality of kinds of derivatives. However, it is preferable that the camptothecin derivatives bonded to chains of one molecular of the block copolymer are identical.

In the polyglutamic acid segment of General Formula (1), glutamic acid units in which a camptothecin derivative is bonded to a side-chain carboxy group, and glutamic acid units in which the above-described **R**₃ group is bonded to a side-chain carboxy group, each independently exist in a random arrangement. Since the **R**₃ group may be a hydroxy group and/or -N(**R**₆)CONH(**R**₇), in the polyglutamic acid segment of the present invention, a glutamic acid unit to which a camptothecin derivative is bonded, a glutamic acid unit to which -N(**R**₆)CONH(**R**₇) moiety is bonded, and a glutamic acid unit of which side chain is a free carboxy group or a salt thereof, each independently exist in a random arrangement.

According to the present invention, the glutamic acid unit to which a camptothecin derivative is bonded is an essential segment constituent. In General Formula (1), the existence amount of the glutamic acid unit to which a camptothecin derivative is bonded is represented by **d,** and the existence amount occupies 1% to 100% of the total polymerization number of the glutamic acid segment. The existence proportion of the **d** in the polyglutamic acid segment is preferably 20% to 70%. The bonding amount of the camptothecin derivative fixes the content of the active ingredient at the time of using the block copolymer as a pharmaceutical product, and significantly affects the pharmacokinetics in vivo after administration and thereby participates in the exhibition of efficacy or side effects.

On the other hand, the **R**₃ group-bonded glutamic acid unit is an optional segment configuration. That is, a glutamic acid unit to which the camptothecin derivative is not bonded is the **R**₃ group-bonded glutamic acid unit. In General Formula (1), the existence amount of the **R**₃ group-bonded glutamic acid unit is represented by **e**, and the existence amount occupies 0% to 99% of the total polymerization number of the glutamic acid segment. It is preferable that the existence proportion of the **e** in the polyglutamic acid segment is 30% to 80%.

The **R**₃ group is a hydroxy group and/or -N(**R**₆)CONH(**R**₇). The -N(**R**₆)CONH(**R**₇) is an optional substituent, and it is preferable that a glutamic acid unit to which the camptothecin derivative is not bonded has a hydroxy group as a main substituent. The existence proportion of the glutamic acid unit in which **R**₃ is a hydroxy group with respect to the total polymerization number of glutamic acid (**d** + **e**) in the polyglutamic acid segment is preferably 15% to 80%, and the existence proportion of a glutamic acid unit in which **R**₃ is - N(**R**₆)CONH(**R**₇) is 0% to 65%.

Meanwhile, the block copolymer of General Formula (1) of the present invention has a property of forming associated aggregates in an aqueous solution. In order to obtain a stable associated aggregate forming ability, a balance between the hydrophilicity of the polyethylene glycol segment and the hydrophobicity of the polyglutamic acid segment may be set as appropriate. Preferably, a block copolymer in which **t** of the polyethylene glycol segment in General Formula (1) is an integer from 90 to 340 and (**d** + **e**) of the total number of glutamic acid units is an integer from 8 to 40 is used, and a block copolymer in which the existence proportion of **d,** which represents the existence amount of glutamic acid units to which the camptothecin derivative is bonded, in the polyglutamic acid segment is 20% to 70%, is used.

That is, the block copolymer represented by General Formula (1) having a camptothecin derivative bonded thereto is preferably a block copolymer in which the content of glutamic acid units having a camptothecin derivative bonded thereto is 20% to 70%; **R**₃ include a hydroxy group as an essential component; the content of glutamic acid units having a hydroxy group as **R**₃ with respect to the total polymerization number of the polyglutamic acid segment is 15% to 80%; and the content of glutamic acid units having the - N(**R**₆)CONH(**R**₇) group as **R**₃ is 0% to 65%. In a case in which **R**₃ is a hydroxy group, the side-chain carboxy group is a free-form glutamic acid unit. The side-chain carboxylic acid is in a free acid form; however, the side-chain carboxylic acid may also be in the form of a salt that may be used as a pharmaceutical product, and the form of the salt of an alkali metal or alkaline earth metal is also included as the present invention. The salt of an alkali metal or an alkaline earth metal is preferably a salt derived from an additive for pharmaceutical preparation that will be described below.

Next, a method for producing the block copolymer represented by General Formula (1) according to the present invention will be explained by giving an example.

The block copolymer may be produced by coupling a camptothecin derivative having a hydroxy group at the 10-position with a "block copolymer in which a polyethylene glycol segment and a free-form polyglutamic acid segment are linked together" by an esterification reaction. Optionally, by a coupling reaction of N(**R**₆)CONH(**R**₇) group for **R**₃, the block copolymer according to the present invention having a camptothecin derivative bonded thereto may be produced. The methods of the coupling reactions of the camptothecin derivative having a hydroxy group at the 10-position and the optional -N(**R**₆)CONH(**R**₇) group are not particularly limited. A camptothecin derivative having a hydroxy group at the 10-position may be first subjected to a coupling reaction and then the -N(**R**₆)CONH(**R**₇) group may be subjected to a coupling reaction; a reverse process thereof is also acceptable; or it is also acceptable to perform the coupling reactions simultaneously.

The method for constructing the "block copolymer in which a polyethylene glycol segment and a free-form polyglutamic acid segment are linked together" may be any of a method of linking a polyethylene glycol segment to a polyglutamic acid segment; and a method of polymerizing polyglutamic acid successively to a polyethylene glycol segment.

A method for synthesizing the block copolymer represented by General Formula (1) according to the present invention will be explained using an example in which the camptothecin derivative is 7-ethyl-10-hydroxycamptothecin, and the hydroxy group at the 10-position of the camptothecin derivative is bonded to a carboxy group of the glutamic acid segment of the block copolymer through an ester bond. Meanwhile, the camptothecin derivative-bonded block copolymer may be produced by the method disclosed in WO 2004/039869 A. In the following description, the production method described in this literature will be disclosed.

Regarding a method for synthesizing the "block copolymer in which a polyethylene glycol segment and a free-form polyglutamic acid segment are linked together", a method of sequentially reacting N-carbonylglutamic anhydride with a polyethylene glycol compound having one end modified with an alkoxy group and the other end modified with an amino group, and constructing a polyglutamic acid structural part on the one end of the polyethylene glycol segment, may be mentioned. In this case, the N-carbonylglutamic anhydride is preferably a glutamic acid derivative in which the carboxy group of a glutamic acid side chain is modified with an appropriate carboxylic acid protective group. The carboxylic acid protective group is not particularly limited; however, an ester protective group is preferred.

More specifically, a method for producing a block copolymer having a polyethylene glycol segment and a polyglutamic acid segment by sequentially reacting γ-benzyl-N-carbonylglutamic anhydride with a polyethylene glycol compound modified with a methoxy group at one end and modified with an amino group at the other end, and performing successive polymerization, may be mentioned. At this time, the glutamic acid polymerization number of the polyglutamic acid segment may be controlled by adjusting the equivalent of the γ-benzyl-N-carbonylglutamic anhydride to be used.

Subsequently, the benzyl group of the polyglutamic acid segment is deprotected by an appropriate method, and thereby the "block copolymer including a polyethylene glycol segment and a polyglutamic acid segment linked together" may be produced. Examples of the deprotection reaction for a benzyl group include a hydrolysis reaction under alkaline conditions and a hydrogenation reduction reaction.

Next, 7-ethyl-10-hydroxycamptothecin is coupled with the "block copolymer in which a polyethylene glycol segment and a free-form polyglutamic acid segment are linked together" in the co-presence of a carbodiimide condensing agent. By using this method, 7-ethyl-10-hydroxycamptothecin and an - N(**R**₆)CONH(**R**₇) group may be simultaneously bonded to the block copolymer, and therefore, this is an advantageous reaction. Furthermore, in the condensation reaction, the bonding amount of the camptothecin derivative may be controlled by adjusting the equivalent of 7-ethyl-10-hydroxycamptothecin to be used. Furthermore, the amount of introduction of the -N(**R**₆)CONH(**R**₇) group may be controlled by adjusting the equivalent of the carbodiimide condensing agent to be used.

The remaining glutamic acid units in which the side-chain carboxy group is not chemically modified, excluding the glutamic acid units having the camptothecin derivative and the -N(**R**₆)CONH(**R**₇) group bonded thereto, become the glutamic acid units in which **R**₃ is a hydroxy group. The amount of the glutamic acid units having a hydroxy group as **R**₃ may be controlled by adjusting the equivalents of the camptothecin derivative to be used and the carbodiimide condensing agent to be used.

The carbodiimide condensing agent thus used may be used without any particular limitations as long as the carbodiimide condensing agent is a condensing agent capable of ester-bonding the camptothecin derivative to the side-chain carboxy group of a glutamic acid unit. Preferred examples include dicyclohexylcarbodiimide (DCC), diisopropylcarbodiimide (DIPCI), and 1-ethyl-3-(3-dimethylaminopropyl)carbodiimide hydrochloride (WSC). At the time of the condensation reaction, a reaction aid such as N,N-dimethylaminopyridine (DMAP) may also be used. Incidentally, in the case of using DCC as the carbodiimide condensing agent, **R**₆ and **R**₇ each represent a cyclohexyl group; in the case of using DIPCI, **R**₆ and **R**₇ each represent an isopropyl group; and in the case of using WSC, **R**₆ and **R**₇ represent a mixture of an ethyl group and a 3-dimethylaminopropyl group.

The block copolymer having a camptothecin derivative bonded thereto according to the present invention may be synthesized by linking an appropriate amount of 7-ethyl-10-hydroxycamptothecin and an -N(**R**₆)CONH(**R**₇) group as an optional substituent of **R**₃ to a glutamic acid side chain of the "block copolymer in which a polyethylene glycol segment and a free-form polyglutamic acid segment are linked together" by the above-described reaction, and then appropriately subjecting the resultant copolymer to a purification process. It is preferable that residual amine components are removed by a cation exchange resin or the like as the purification process, and also the side-chain hydroxy group form of polyglutamic acid is prepared into a free acid form.

The block copolymer represented by General Formula (1) having a camptothecin derivative bonded thereto has a performance of slowly dissociating the camptothecin derivative in a phosphate buffer physiological saline (PBS) solution and continuously releasing the camptothecin derivative. For example, in a case in which the camptothecin derivative is 7-ethyl-10-hydroxycamptothecin and is bonded through an ester-bond formed by the hydroxy group at the 10-position, when this is administered in vivo, the block copolymer has a property of slowly releasing 7-ethyl-10-hydroxycamptothecin.

Low molecular weight drugs that are clinically used in general exhibit the maximum blood concentration of the drug immediately after administration and are subsequently excreted from the body relatively rapidly. In contrast, since the camptothecin derivative-bonded block copolymer slowly dissociates 7-ethyl-10-hydroxycamptothecin as an active ingredient, the camptothecin derivative-bonded block copolymer is a preparation that does not significantly increase the blood concentration of the active ingredient in blood after administration and exhibits a sustained blood concentration profile.

Furthermore, since the camptothecin derivative-bonded block copolymer includes the polyglutamic acid segment having a hydrophobic camptothecin derivative, the copolymer has associative properties based on a hydrophobic interaction between the polyglutamic acid segments in an aqueous solution. Meanwhile, the polyethylene glycol segment in the block copolymer is hydrophilic. Therefore, the block copolymer in an aqueous solution forms core-shell type micelle-like associated bodies in which hydrophobic polyglutamic acid segments form a core part based on associated aggregates, and hydrophilic polyethylene glycol segments cover the periphery of the core part to form an outer shell and thereby form a shell layer.

For the micelle-like associated bodies, the associated body-forming property may be evaluated by light scattering intensity measurement using laser light or the like. For example, the light scattering intensity may be used directly as a property value for the associated aggregate-forming property. An aqueous solution of the block copolymer usually shows several ten thousand to several hundred thousand cps as the light scattering intensity value, and it is acknowledged that the block copolymer forms associated aggregates.

Furthermore, the total molecular weight of the associated aggregates may be calculated from the light scattering intensity, using high molecular weight standard products of polyethylene glycol or the like as a reference. When the block copolymer is allowed to form associated aggregates in an aqueous solution, it may be calculated from the results of a light scattering intensity analysis that the associated aggregates are associated aggregates having a total molecular weight of several millions or more. Therefore, it may be conceived that the micelle-like associated bodies are formed by association of a plurality of molecules, such as several dozens to several hundred molecules, of the block copolymer.

Furthermore, according to a particle size analysis by a dynamic light scattering analysis, the block copolymer in an aqueous solution has a property of forming nanoparticulate bodies having a particle size of several nanometers to several hundred nanometers.

The block copolymer that forms nanoparticles as associated aggregates in an aqueous solution is distributed in the body, when administered in vivo, in the state of the associated nanoparticles in blood. High molecular weight compounds or nanoparticle-like objects show significantly different pharmacokinetic behavior in vivo or tissue distribution compared to those of low molecular weight drugs that are conventionally used. Therefore, it is known that the in vivo retentivity and tissue distribution of the camptothecin derivative-bonded block copolymer that forms associated nanoparticles are determined depending on the molecular weight or particle size of the associated nanoparticles, and that the block copolymer particularly stays and is distributed in tumor tissues. From this, the camptothecin derivative-bonded block copolymer is an antitumor preparation which has completely different characteristics from conventional low molecular weight camptothecin preparations in pharmaceutical efficacy exhibitions and side-effects exhibitions, and which is capable of providing a new clinical therapeutic method of using the camptothecin derivative.

Therefore, it is important that the block copolymer forms controlled nanoparticles to have a desired molecular weight and particle size based on its specific associative property, whereby preferable pharmacokinetics and tissue distribution are obtained as an antitumor agent. The formation of associated nanoparticles having a desired molecular weight and particle size may be considered as an important product quality management item in order to exhibit the performance. Furthermore, suppression of the formation of the released camptothecin derivative by cleaving a bond between the block copolymer and the camptothecin derivative contributes to reduced exhibition of side effects, and the suppression may also be similarly considered as an important product quality management item.

Regarding the block copolymer, the physical stability related to the associated aggregate-forming property and the chemical stability related to the suppression of production of the released camptothecin derivative are important as product quality management items.

The present invention relates to a pharmaceutical preparation including the block copolymer represented by General Formula (1) as an active ingredient, and one or more additives selected from the group consisting of arginine, histidine, and sodium chloride. The present invention may provide a pharmaceutical preparation that maintains the associated aggregate-forming property of the block copolymer during storage of the pharmaceutical preparation by using arginine, histidine, and sodium chloride as additives for a pharmaceutical preparation, and/or secures physical and chemical stability of suppressing the bond cleavage of the camptothecin derivative.

The histidine, arginine, and sodium chloride thus used may be used without any particular limitations as long as they have the purity required for the use in pharmaceutical preparations. Histidine and arginine may be L-forms, may be D-forms, or may be a mixture of these. Histidine, arginine, and sodium chloride may be used alone, or two or more kinds thereof may be used as a mixture.

The content of the histidine, arginine, and sodium chloride used as additives is preferably from 10 parts by mass to 500 parts by mass with respect to 1 part by mass of the content of the camptothecin derivative in the block copolymer represented by General Formula (1). When the content of the additives is smaller than 10-fold parts by mass of the content of the bonded camptothecin derivative, there is a risk that the effects of maintaining the associative property and/or chemical stability of the block copolymer may not be sufficiently obtained. Meanwhile, from the viewpoint of a stabilization effect, the upper limit of the use amount of the additives is not particularly limited; however, it is preferable that the upper limit is set to about 500 parts by mass, in view of the appropriateness of the dose as a pharmaceutical preparation. It is more preferable that the additives are used in an amount of 25 to 100 parts by mass with respect to 1 part by mass of the content of the bonded camptothecin derivative.

The pharmaceutical preparation of the present invention may provide, by using the histidine, arginine, and sodium chloride as additives, a pharmaceutical preparation which has excellent stability with regard to the associated body-forming rate analyzed in an aqueous solution even if the pharmaceutical preparation is stored under light-shielded conditions for four weeks at 40°C, and/or in which the formation of the released camptothecin derivative by cleaving a bond is suppressed.

The pharmaceutical preparation of the present invention including a block copolymer having a camptothecin derivative bonded thereto as an active ingredient is desirably such that when the pharmaceutical preparation is prepared so that a camptothecin derivative content concentration in an aqueous solution is 1 mg/mL, the pH of the aqueous solution is 3.0 to 5.0.

In order to adjust the pH of the aqueous solution of the pharmaceutical preparation of the present invention to 3.0 to 5.0, a pH adjusting agent may be used in the pharmaceutical preparation. Regarding the pH adjusting agent, any acid that may be used as a pharmaceutical product additive may be used without any particular limitations, and examples include hydrochloric acid, sulfuric acid, phosphoric acid, citric acid, tartaric acid, malic acid, mesylic acid, tosylic acid, and besylic acid. Buffer agents including these acidic additives as main components and further including an alkali metal salt, an alkaline earth metal salt, and an ammonium salt, may also be used. Preferred examples of the acid additive include hydrochloric acid, phosphoric acid, citric acid, and tartaric acid, and it is preferable that these acids are used in an appropriate amount of addition so that the pH of the aqueous solution of the pharmaceutical preparation is set to 3.0 to 5.0. A pharmaceutical preparation in which hydrochloric acid, phosphoric acid, citric acid, or tartaric acid is used as a pH adjusting agent and the pH has been adjusted to 3.0 to 5.0, is more preferred.

The pharmaceutical preparation of the present invention is preferably a preparation that is intravascularly administered for injection or infusion, and is preferably an injectable preparation that may be intravenously administered. The preparation form is preferably a preparation form such as a freeze-dried preparation, an injectable formulation that may be prepared into an injectable solution by diluting the preparation at the time of use, or a diluted solution preparation that may be administered directly. In a case in which the pharmaceutical preparation of the present invention is administered as a pharmaceutical product, the pharmaceutical preparation is usually used as a solution of the pharmaceutical preparation by using water, physiological saline, a 5% aqueous solution of glucose or mannitol, a water-soluble organic solvent, or the like. Examples of the water-soluble organic solvent include single solvents such as glycerol, ethanol, dimethyl sulfoxide, N-methylpyrrolidone, polyethylene glycol, and Cremophor; and mixed solvents of these.

It is preferable that the pharmaceutical preparation of the present invention is a freeze-dried preparation when the associated body-forming stability and chemical stability of the block copolymer represented by General Formula (1) are considered.

The pharmaceutical preparation of the present invention may include other pharmacologically acceptable additives that are usually used. Examples of the other additives that may be used include a binder, a lubricating agent, a disintegrant, a solvent, an excipient, a solubilizing agent, a dispersant, a stabilizer, a suspending agent, a preservative, a soothing agent, a colorant, and a fragrance.

As other additives for the pharmaceutical preparation of the present invention, it is preferable to use sugars, polyols, polyethylene glycols, other amino acids, inorganic salts, and the like.

Sugars in pharmaceutical preparations also generally function as excipients, and the sugars according to the present invention are also used as excipients.

Examples of the sugars include arabinose, isomaltose, galactosamine, galactose, xylose, glucosamine, glucose, gentiobiose, kojibiose, sucrose, cellobiose, sophorose, thioglucose, turanose, deoxyribose, trehalose, nigerose, palatinose, fucose, fructose, maltose, mannose, melibiose, lactose, rhamnose, and laminaribiose.

Examples of the polyols include xylytol, sorbitol, maltitol, mannitol, and meglumine.

Examples of the polyethylene glycols include polyethylene glycol 300, polyethylene glycol 400, polyethylene glycol 600, and polyethylene glycol 4000.

Examples of the other amino acids include glycine, proline, aspartic acid, glutamic acid, serine, and lysine hydrochloride.

Examples of the inorganic salts include calcium chloride, calcium oxide, and magnesium sulfate.

The other additives to be used may be used without any particular limitations as long as the additives have the purity that may be used in pharmaceutical preparations. These may be used singly or may be used as mixtures thereof. Regarding the other additives, it is preferable to use inositol, glucose, trehalose, fructose, maltose, mannitol, lactose, and sucrose.

In regard to the pharmaceutical preparation of the present invention, in the case of using other optional additives, it is preferable that the optional additives are used in an amount of 0.1 to 50 parts by mass with respect to 1 part by mass of the content of the bonded camptothecin derivative in the block copolymer. More preferably, it is desirable that the additives are used in an amount of 0.5 to 30 parts by mass, and it is particularly preferable that the additives are used in an amount of 1 to 25 parts by mass.

The pharmaceutical preparation of the present invention is preferably an intravascularly administered preparation such as an injectable preparation or an infusible preparation, and is preferably a preparation form such as a freeze-dried preparation or an injectable formulation.

In the case of producing the pharmaceutical preparation into a freeze-dried preparation, the block copolymer having a camptothecin derivative bonded thereto as a pharmaceutically active ingredient is prepared into an aqueous solution together with optional additives for formulation, and a medical solution is prepared by adjusting the pH of the aqueous solution. This is preferably subjected to sterilization by filtration, subsequently dispensed into preparation vials, and freeze-dried, and thereby a freeze-dried preparation may be prepared. For the adjustment of pH, a pH adjusting agent may be used, or pH adjustment may also be carried out with the active ingredient itself by using the camptothecin derivative-bonded block copolymer containing a glutamic acid unit in which the side chain is a free carboxylic acid, as an active ingredient.

On the other hand, in the case of producing the pharmaceutical preparation into an injectable formulation, an aqueous solution is prepared with the block copolymer together with optional additives for formulation. Subsequently, the aqueous solution is prepared into a medical solution having its pH adjusted, and this is preferably subjected to sterilization by filtration and then dispensed into preparation vessels. Thus, an injectable formulation may be prepared. For the adjustment of pH, a pH adjusting agent may be used, or pH adjustment may be carried out using the active ingredient itself.

The present invention is an invention related to a pharmaceutical preparation including the block copolymer having a camptothecin derivative bonded thereto as an active ingredient, the pharmaceutical preparation being a pharmaceutical unit preparation having the block copolymer filled into a predetermined preparation form at an arbitrary content.

In a case in which a pharmaceutical preparation is prepared, usually, a pharmaceutical formulation that is durable for a certain time period upon long-term storage is examined in consideration of the chemical stability of the active ingredient, by using pharmaceutically acceptable additives. In the case of a pharmaceutical preparation including the block copolymer of the present invention having a camptothecin derivative bonded thereto as an active ingredient, since the nanoparticle-forming property obtainable when the pharmaceutical preparation is prepared into an aqueous solution is an important product quality management item, it is necessary to formulate a preparation also in consideration of the stability of the nanoparticle-forming property.

In regard to the associated body-forming property of the block copolymer having a camptothecin derivative bonded thereto in the pharmaceutical preparation of the present invention, for example, the associated body-forming property may be evaluated using a measuring instrument capable of measuring the laser scattered light intensity, by taking the scattered light intensity as an index. Usually, the pharmaceutical preparation of the present invention is recognized as associated aggregates because a measured value of several ten thousand to several hundred thousand cps is obtained as the scattered light intensity value by a method for measuring the static light scattering intensity.

Regarding the measuring instrument for a scattered light intensity analysis, for example, measurement may be made using a dynamic light scattering photometer manufactured by Otsuka Electronics Co., Ltd., DLS-8000DL.

Regarding a method for evaluating the stability of the pharmaceutical preparation of the present invention, the change ratio for the associated body-forming property in the case of storing the pharmaceutical preparation under light-shielded conditions for four weeks at 40°C may be used as an index. In an evaluation method of using a light scattering intensity analysis, evaluation may be carried out by employing the change ratio for forming associated bodies by comparing the light scattering intensity with the initial value obtained before a storage test. When the pharmaceutical preparation is stored under light-shielded conditions for four weeks at 40°C, the change ratio for the associated body-forming property based on the molecular weight of the associated bodies as an index is 10% or less. That is, in a case in which the associated body-forming property is markedly deteriorated during storage of the preparation and the initial associated nanoparticles may not be formed, there is a problem that the effectiveness of the camptothecin derivative-bonded block copolymer is decreased. Therefore, it is desirable that the pharmaceutical preparation is a preparation in which the associated body-forming property is not deteriorated in the state of being stored as a pharmaceutical preparation. In regard to the test method described above, it is preferable that the change ratio for the associated form-forming property based on the molecular weight of the associated bodies as an index is 10% or less. Meanwhile, the change ratio of the molecular weight of the associated bodies is represented as an absolute value of the change ratio of the value obtained after storage for four weeks at 40°C with respect to the initial value.

Furthermore, regarding a method for evaluating the stability of the pharmaceutical preparation of the present invention, the amount of the released camptothecin derivative produced by cleavage of the camptothecin derivative bonded to the glutamic acid side chain in a case in which the pharmaceutical preparation is stored under light-shielded conditions for four weeks at 40°C, may be employed as an index. The production amount of the released camptothecin derivative may be quantitatively analyzed by an HPLC method. Since the released camptothecin derivative acts as an active ingredient, when the production amount thereof is large, there is a risk that the pharmacokinetics based on the block copolymer may not be obtained, and the desired efficacy may not be exhibited. Accordingly, suppression of the production amount of the released camptothecin derivative is an important management item for the pharmaceutical product.

When the change ratio of the camptothecin derivative release rate obtained by employing the release of the camptothecin derivative of the block copolymer as an index is evaluated, it is preferable that in a case in which the pharmaceutical preparation is stored under light-shielded conditions for four weeks at 40°C, the change ratio for the bonding of the camptothecin derivative to the block copolymer is 7.0 times or less, and more preferably 5.0 times or less. The camptothecin derivative release rate is represented by the ratio of the camptothecin derivative release rate after storage for four weeks at 40°C with respect to the initial camptothecin derivative release rate.

The pharmaceutical preparation of the present invention may be utilized as a pharmaceutical product including a conjugated camptothecin derivative as an active ingredient. Particularly, it is preferable to use the pharmaceutical preparation as an antitumor agent for cancer chemotherapy.

The use of the pharmaceutical preparation of the present invention is not particularly limited as long as the pharmaceutical preparation is used for carcinomas for which the camptothecin derivative provides a therapeutic effect; however, specific examples include small cell lung cancer, non-small cell lung cancer, cervical cancer, ovarian cancer, stomach cancer, colorectal cancer, breast cancer, squamous cell carcinoma, malignant lymphoma, infant malignant solid tumor, pancreatic cancer, and multiple myeloma.

The dose of the pharmaceutical preparation of the present invention may be definitely varied depending on the gender, age, physiological condition, pathological condition, and the like of the patient; however, the pharmaceutical preparation is usually administered parenterally at a dose of 0.01 to 500 mg/m² (body surface area), and preferably 0.1 to 250 mg/m²), in terms of the camptothecin derivative, per day for an adult. Administration by injection is preferably carried out through a vein, an artery, a lesion (for example, a tumor tissue), or the like.

### EXAMPLES

### [Synthesis Example 1]

Synthesis of 7-ethyl-10-hydroxycamptothecin-bonded block copolymer (Compound 1) in which in General Formula (1), **R**₁ = methyl group, **R**₂ = acetyl group, **A** = trimethylene group, **R**₆ = **R**₇ = isopropyl group, **d** + **e** = 24, **t** = 273, proportion of **d** with respect to **d** + **e** is 44%, and proportion of **e** is 56% (percentage content of glutamic acid unit having hydroxy group for **R**₃ is 30%, and percentage content of glutamic acid unit having -N(**R**₆)CONH(**R**₇) for **R**₃ is 26%)

Compound 1 was synthesized based on the description of WO 2004/39869 A. That is, Compound 1 was obtained by reacting 7-ethyl-10-hydroxycamptothecin (EHC) with a methoxy polyethylene glycol-polyglutamic acid block copolymer (a block copolymer including a methoxy polyethylene glycol structural moiety having a molecular weight of 12 kilodaltons and having a methyl group at one end and aminopropyl group at the other end; a polyglutamic acid structural moiety having the N-terminal modified with an acetyl group and having a polymerization number of 24; and a trimethylene group as a linking group), using diisopropylcarbodiimide (DIPCI) and N,N-dimethylaminopyridine (DMAP), subsequently treating the reaction product with an ion exchange resin (DOWEX 50 (H+) manufactured by Dow Chemical Company), and freeze-drying the resultant.

Compound 1 thus obtained was hydrolyzed for 10 minutes at room temperature using an aqueous solution of sodium hydroxide, and then released EHC was quantitatively analyzed by an HPLC method to determine the EHC content. The EHC content was 21.0% by mass.

### [Example 1]

A medical solution including Compound 1 at a content of 1 mg/mL in terms of EHC and arginine at a content of 50 mg/mL was prepared using water for injection. The pH of the solution was adjusted to 4 using phosphoric acid, and then the solution was sterilized by filtration. The filtered medical solution was charged into glass vials in an amount of 3 mL each and was freeze-dried. Subsequently, the glass vials were tightly sealed with rubber stoppers, and it was designated as Example 1.

### [Example 2]

A medical solution was prepared by an operation similar to the production method of Example 1, except that histidine was used at a content of 50 mg/mL instead of arginine, and the medical solution was freeze-dried. This freeze-dried preparation was tightly sealed with rubber stoppers, and it was designated as Example 2.

### [Example 3]

A medical solution was prepared by an operation similar to the production method of Example 1, except that sodium chloride was used at a content of 50 mg/mL instead of arginine, and the medical solution was freeze-dried. This freeze-dried preparation was tightly sealed with rubber stoppers, and it was designated as Example 3.

### [Comparative Example 1]

A medical solution including Compound 1 at a content of 1 mg/mL in terms of EHC was prepared using water for injection. The pH of the solution was adjusted to 4 using phosphoric acid, and then the solution was sterilized by filtration. The filtered solution was charged into glass vials in an amount of 3 mL each and was freeze-dried. Subsequently, the glass vials were tightly sealed with rubber stoppers, and it was designated as Comparative Example 1.

### [Comparative Example 2]

A medical solution was prepared by an operation similar to the production method of Example 1, except that glycine was used at a content of 50 mg/mL instead of arginine, and the medical solution was freeze-dried. This freeze-dried preparation was tightly sealed with rubber stoppers, and it was designated as Comparative Example 2.

### [Comparative Example 3]

A medical solution was prepared by an operation similar to the production method of Example 1, except that proline was used at a content of 50 mg/mL instead of arginine, and the medical solution was freeze-dried. This freeze-dried preparation was tightly sealed with rubber stoppers, and it was designated as Comparative Example 3.

### [Comparative Example 4]

A medical solution was prepared by an operation similar to the production method of Example 1, except that polyethylene glycol 4000 was used at a content of 50 mg/mL instead of arginine, and the medical solution was freeze-dried. This freeze-dried preparation was tightly sealed with rubber stoppers, and it was designated as Comparative Example 4.

### Test Example 1: Changes in light scattering intensities of associated aggregates of Examples and Comparative Examples under storage conditions of 40°C/four weeks

3 mL of water for injection was added to each of the preparations of Examples 1 to 3 and Comparative Examples 1 to 4 obtained immediately after freeze-drying, and solutions of the respective preparations at an EHC content of 1 mg/mL were prepared. For each of these solutions, the pH was measured. Subsequently, 3 mL of water for injection was further added to the solution. The amount of scattered light of the associated aggregates in this solution was measured by a static light scattering method (SLS method). This was designated as the initial amount of scattered light. The measuring instruments and measurement conditions are shown below.

Separately, the freeze-dried preparations of Examples 1 to 3 and Comparative Examples 1 to 4 were stored under light-shielded conditions for four weeks at 40°C. Subsequently, 3 mL of water for injection was added to each of the respective Examples and Comparative Examples, and the solution pH was measured. Subsequently, samples similar to the initial samples were prepared, and the amount of scattered light of the associated aggregates in each of the solutions of the freeze-dried preparations was measured.

The measurement results for the solution pH and the amount of scattered light of the initial samples and the solution pH and the amount of scattered light after storage at 40°C/four weeks, and the change ratios of the amount of scattered light for each of the samples are summarized in Table 1.

### Measuring instruments and measurement conditions

Light scattering photometer: DLS-8000 DL (manufactured by Otsuka Electronics Co., Ltd.)
Contra roller: LS-81 (manufactured by Otsuka Electronics Co., Ltd.)
Pump contra roller: LS-82 (manufactured by Otsuka Electronics Co., Ltd.)
High-sensitivity differential refractometer: DRM-3000 (manufactured by Otsuka Electronics Co., Ltd.)
Circulating constant-temperature tank: LAUDA E200
Wavelength: 632.8 nm (He-Ne)
Angle: 90°
Ph1: OPEN
Ph2: SLIT
ND Filter: 10%
Dust cutoff setting: 10

**[Table 1]**

| | pH upon redissolution | | Amount of light (cps) | | Change ratio of amount of scattered light (%) |
|---|---|---|---|---|---|
| | Initial | 40°C/4w | Initial | 40°C/4w | |
| Example 1 | 4.0 | 4.0 | 15323 | 15110 | 1.4 |
| Example 2 | 4.0 | 4.0 | 18475 | 19213 | 4.0 |
| Example 3 | 4.3 | 4.3 | 23138 | 23170 | 0.1 |
| Comparative Example 1 | 4.1 | 4.1 | 5268 | 3710 | 29.6 |
| Comparative Example 2 | 4.0 | 4.1 | 17493 | 14589 | 16.6 |
| Comparative Example 3 | 4.0 | 4.1 | 18668 | 16070 | 13.9 |
| Comparative Example 4 | 4.2 | 4.2 | 17428 | 15146 | 13.1 |

Measurement of static light scattering of a sample solution involves analysis of the state in which the block copolymer (Compound 1) in each aqueous solution undergoes self-association and forms associated aggregates, and the amount of scattered light represents the extent of formation of the associated aggregates.

Each sample was stored under the conditions of 40°C/four weeks according to Test Example 1, and the amount of scattered light of the associated aggregates in the solution of each freeze-dried preparation was measured. As a result, it was made clear that in Examples 1 to 3, even if the storage time for storage under the conditions of 40°C/four weeks in solutions is passed, there is little change in the amount of scattered light, and no change occurs in the associated aggregate-forming property. On the other hand, in Comparative Examples 1 to 4, the amount of scattered light changed by 13.1% to 29.6% after storage under the conditions of 40°C/four weeks, and thus, it is suggested that changes occur in the associated aggregate-forming property.

From this, it was recognized that when arginine, histidine, or sodium chloride is used as an additive in a pharmaceutical preparation including a block copolymer (Compound 1) as an active ingredient, a stable preparation in which there is little change in the associated aggregate-forming property of the camptothecin derivative-bonded block copolymer as an active ingredient during the storage of the preparation, may be prepared.

### Test Example 2: Preparation stability test (camptothecin derivative release ratio)

The freeze-dried preparations of Example 1 to 3 and Comparative Examples 1 to 4 were stored under light-shielded conditions for four weeks at 40°C. Subsequently, released EHC of each pharmaceutical composition was quantitatively analyzed by an HPLC method, and thus, the preparation stability was evaluated. The results are summarized in Table 2. The extent of released EHC is represented as the released EHC change ratio, which is obtained by dividing the amount of released EHC after storage for four weeks at 40°C by the initial value. The measurement results are summarized in Table 2.

**[Table 2]**

| | pH upon redissolution | | EHC release ratio (%) | | Change ratio of EHC release rate |
|---|---|---|---|---|---|
| | Initial | 40°C/4w | Initial | 40°C/4w | |
| Example 1 | 4.0 | 4.0 | 0.24 | 1.05 | 4.38 |
| Example 2 | 4.0 | 4.0 | 0.30 | 1.08 | 3.60 |
| Example 3 | 4.3 | 4.3 | 0.17 | 0.41 | 2.41 |
| Comparative Example 1 | 4.1 | 4.1 | 0.37 | 2.68 | 7.24 |
| Comparative Example 2 | 4.0 | 4.1 | 0.21 | 6.44 | 30.67 |
| Comparative Example 3 | 4.0 | 4.1 | 0.33 | 7.50 | 22.73 |
| Comparative Example 4 | 4.2 | 4.2 | 0.22 | 5.50 | 25.00 |

As a result of Test Example 2, in Examples 1 to 3, the production amount of released EHC was 2.41 to 4.38 times. In contrast, in Comparative Examples 1 to 4, the production amount of released EHC was as large as 7.24 to 30.67 times. Thus, it was found that the EHC bonded to the block copolymer was cleaved and released under the storage conditions. From this, it was found that when arginine, histidine, or sodium chloride is used as an additive in a pharmaceutical preparation including a block copolymer (Compound 1) as an active ingredient, a pharmaceutical preparation including a chemically stable polymerized camptothecin derivative, in which EHC dissociation is suppressed, may be provided.

From the results of Test Examples 1 and 2, it was found that when arginine, histidine, or sodium chloride is used as an additive in a pharmaceutical preparation including a block copolymer conjugated with a camptothecin derivative as an active ingredient, a pharmaceutical preparation having excellent associated aggregate-forming property and excellent chemical stability may be provided.

## Claims

1. A pharmaceutical preparation comprising:
a block copolymer of a polyethylene glycol segment and a polyglutamic acid segment linked together, the polyglutamic acid segment comprising a glutamic acid unit having a camptothecin derivative bonded thereto; and
one or more additives selected from the group consisting of arginine, histidine, and sodium chloride,
wherein the block copolymer is a block copolymer represented by General Formula (1): 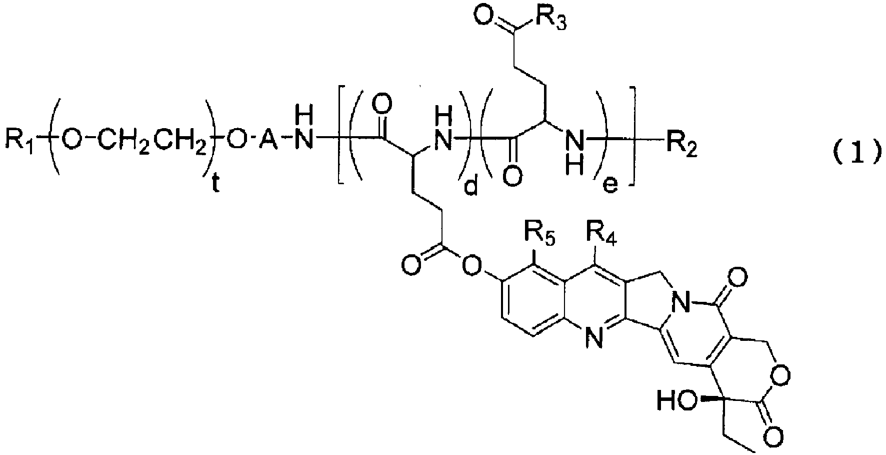 wherein
**R₁** represents a hydrogen atom or an optionally substituted (C1-C6) alkyl group;
**A** represents a (C1-C6) alkylene group;
**R₂** represents any one selected from the group consisting of a hydrogen atom, an optionally substituted (C1-C6) acyl group, and an optionally substituted (C1-C6) alkoxycarbonyl group;
**R₃** represents a hydrogen group and/or -N(**R₆**)CONH(**R₇**);
**R₆** and **R₇**, which may be identical or different, each represent a (C1-C8) alkyl group which may be substituted with a tertiary amino group;
**R₄** represents any one selected from the group consisting of a hydrogen atom, an optionally substituted (C1-C6) alkyl group, and an optionally substituted silyl group;
**R₅** represents a hydrogen atom or an optionally substituted (C1-C6) alkyl group;
**t** represents an integer from 45 to 450;
**d** and **e** each represent an integer; **d** + **e** represents an integer from 6 to 60; the proportion of **d** with respect to **d** + **e** is 1% to 100%; the proportion of **e** is 0% to 99%; and
the polyglutamic acid segment has a polyglutamic acid segment structure in which glutamic acid units having a camptothecin derivative bonded thereto and glutamic acid units having a **R₃** group bonded thereto are each independently arranged randomly.

2. The pharmaceutical preparation according to claim 1, wherein the pharmaceutical preparation is a freeze-dried preparation.
